# EUROPEAN PATENT APPLICATION

(11) **EP 4 454 579 A1**
(43) Date of publication of application: **30.10.2024**
(21) Application number: 24172337.8
(22) Date of filing: 25.04.2024
(51) Int. Cl.: A61B 17/70

(54) **LAMINOPLASTY IMPLANT**

(30) Priority: 27.04.2023 US 202318308067
(71) Applicant: Jenkins III, Arthur L, Riverside Connecticut, CT 06878 (US)
(72) Inventor: Jenkins III, Arthur L, Riverside Connecticut, CT 06878 (US)
(74) Representative: Swea IP Law AB

(57) **Abstract**

An implant (10) for connecting a lamina (130) having an edge (160) separated from a lateral mass (120) of a vertebra (140) during a laminoplasty procedure is provided. The implant has a first portion (20) configured to engage with the lateral mass. The first portion includes a transverse opening (74) for receiving a fastener (76) for fixing the first portion to the lateral mass. The implant includes a second portion (60) configured to engage with the edge of the lamina separated from the lateral mass during the laminoplasty procedure. The second portion being connected to the first portion so that the second portion is rotatable relative to the first portion about an axis of rotation (59). The second portion includes a transverse opening for receiving a fastener. The implant is configured so that the first portion can be secured to the lateral mass prior to separating the lamina therefrom.

## Description

### TECHNICAL FIELD

The present disclosure generally relates to surgical implant. More specifically, the present invention relates to a laminoplasty implant.

### BACKGROUND

In certain pathologies, the spinal canal extending through a patient's vertebrae is or becomes too narrow and constricts the spinal cord extending therethrough. The narrowing may be congenital, potentially affecting patients at any age. Narrowing can also be attributable to other causes, such as age, injury or removal of a spinal disk.

A condition associated with aging, for instance, is spondylolsis, in which intervertebral disc loose water and become less dense. These degenerative changes near the disk can cause an overgrowth of the bone, producing bony spurs called, "osteophytes" that can compress the spinal cord. The constriction of the spinal cord in the cervical spine, for example, often produces pain, weakness, or loss of feeling in extremities. Other causes for narrowing of the spinal canal include disc shrinkage, which causes the disc space to narrow and the annulus to bulge and mushroom out, resulting in pressure on the spinal cord. Degenerative arthritis of facet joints can cause joints to enlarge, or the vertebra to slip with respect to each other, also compressing the spinal cord. Instability between vertebra, such as caused by stretched and thickened ligaments' can also produce pressure on the spinal cord and nerve roots.

Myelopathy, or malfunction of the spinal cord, occurs due to its compression. The rubbing of the spine against the cord can also contribute to this condition, and the spinal cord compression can ultimately compromise the blood vessels feeding the spinal core, further aggravating they myelopathy.

Traditional procedures for decompressing the spinal cord include a laminectomy, in which the lamina and spinal processes are removed to expose the dura covering the spinal cord. Another known procedure is a laminoplasty, in which the lamina is lifted off the dura, but not completely removed. Typically, one side of the lamina is cut, while a partial cut is made on the other side to hinge the lamina away from the spinal cord to increase the size of the spinal canal. A laminoplasty plate is then screwed to a facet and to the hinged open lamina. The plate of an appropriate size is selected and bent to the desired shape and preferably has a plurality of screw holes. A strut of bone can be placed in the open portion within the lamina and the facet to help hold the open position of the lamina. Prior to the operation, the surgeon needs to measure the vertebra to determine the size of the plate necessary for implantation. At that point, a plate can be selected with the appropriate dimensions, and implanted at the site.

A laminoplasty implant is needed that preferably has a uniform size and that allows for the extent of the opening of the lamina relative to the lamina mass to be varied as need to the anatomy of the patient, the position of the cut, and other factors.

A laminoplasty implant is needed that preferably has can be configured to collapse for introduction to the surgical site via a cannula or the like.

Another need, is a laminoplasty implant that can be surgically implant using minimally invasive techniques.

### SUMMARY OF THE INVENTION

The needs set forth herein as well as further and other needs and advantages are addressed by the present teachings, which illustrate solutions and advantages described below.

The present invention resides in one aspect in an implant for connecting a lamina having an edge separated from a lateral mass of a vertebra during a laminoplasty procedure. The implant includes a first portion configured to engage with the lateral mass. The first portion extends defines a surface for interfacing with the lateral mass. The first portion includes a transverse opening for receiving a fastener for fixing the first portion to the lateral mass. The implant includes a second portion configured to engage with the edge of the lamina separated from the lateral mass during the laminoplasty procedure. The second portion extending between a proximal end and a distal end. The proximal end of the second portion is connected to the first portion so that the second portion is rotatable relative to the first portion about an axis of rotation. The second portion defines a transverse opening for receiving a fastener for fixing the second portion to the lamina.

In yet a further embodiment of the implant for connecting a lamina having an edge separated from a lateral mass of a vertebra during a laminoplasty procedure the second portion comprises a receiving section (90) configured to receive the edge of the separated lamina.

In yet a further embodiment of the implant for connecting a lamina having an edge separated from a lateral mass of a vertebra during a laminoplasty procedure, the receiving section (90) is at the distal end of the second portion.

In yet a further embodiment of the implant for connecting a lamina having an edge separated from a lateral mass of a vertebra during a laminoplasty procedure the receiving section includes an outer member extending distally from the second portion The receiving section further includes an inner member extending distally from the second portion. The receiving section defines a space between the outer member and the inner member.

In yet a further embodiment of the implant for connecting a lamina having an edge separated from a lateral mass of a vertebra during a laminoplasty procedure, the receiving section is configured to receive the edge of the lateral mass between the outer member and the inner member.

In yet a further embodiment of the implant for connecting a lamina having an edge separated from a lateral mass of a vertebra during a laminoplasty procedure, the transverse opening for receiving a fastener for fixing the second portion to the lamina extends through the outer member.

In yet a further embodiment of the implant for connecting a lamina having an edge separated from a lateral mass of a vertebra during a laminoplasty procedure, the receiving section defines a transition between the outer member and the inner member, the transition extending along an axis parallel to a vertebral column when the first portion is fixed to the lateral mass.

In yet a further embodiment of the implant for connecting a lamina having an edge separated from a lateral mass of a vertebra during a laminoplasty procedure, a distance between the outer member and the inner member is greater than a width of the lamina at determined at the edge thereof.

In yet a further embodiment of the implant for connecting a lamina having an edge separated from a lateral mass of a vertebra during a laminoplasty procedure, the inner member is configured to abut an inside surface of the lamina and the outer member is configured to abut an outside surface of the lamina when the edge of the lamina is received in the receiving section.

In yet a further embodiment of the implant for connecting a lamina having an edge separated from a lateral mass of a vertebra during a laminoplasty procedure, the inner member and the outer member define a U-shaped configuration for receiving the edge of the lamina.

In yet a further embodiment of the implant for connecting a lamina having an edge separated from a lateral mass of a vertebra during a laminoplasty procedure, the outer member extends from the second portion further than the inner member.

In yet a further embodiment of the implant for connecting a lamina having an edge separated from a lateral mass of a vertebra during a laminoplasty procedure, the transverse opening is positioned in the outer member so that the fastener received through the opening and into the lamina does not interfere with the first protrusion.

In yet a further embodiment of the implant for connecting a lamina having an edge separated from a lateral mass of a vertebra during a laminoplasty procedure, the outer member comprises a flexible portion extending in the direction of the vertebral column. The flexible portion is positioned between the transverse opening in the second portion and the proximal end of the second portion.

In yet a further embodiment of the implant for connecting a lamina having an edge separated from a lateral mass of a vertebra during a laminoplasty procedure, the implant includes a bone fusion material disposed on one or more surfaces of the implant that interface with the bone. The bone fusion material selected to encourage osteointegration between the bone and the implant.

In yet a further embodiment of the implant for connecting a lamina having an edge separated from a lateral mass of a vertebra during a laminoplasty procedure the first portion comprises a plurality of positioning pins extending from the interface surface, The positioning pins are configured to engage with the lateral mass and inhibit movement between the first portion relative to the lateral mass while the fastener is installed.

In yet a further embodiment of the implant for connecting a lamina having an edge separated from a lateral mass of a vertebra during a laminoplasty procedure, the first portion and the axis of rotation are configured so that the first portion can be fixed to the lateral mass prior to cutting the lamina adjacent thereto.

In yet a further embodiment of the implant for connecting a lamina having an edge separated from a lateral mass of a vertebra during a laminoplasty procedure, the axis of rotation extends along an axis parallel to a vertebral column when the first portion is fixed to the lateral mass.

In yet a further embodiment of the implant for connecting a lamina having an edge separated from a lateral mass of a vertebra during a laminoplasty procedure, the implant comprises a hinge between the first portion and the second portion. The hinge facilitates rotation of the first portion and the second portion.

In yet a further embodiment of the implant for connecting a lamina having an edge separated from a lateral mass of a vertebra during a laminoplasty procedure, the receiving section is U-shaped.

These and other aspects of the present invention will become apparent in light of the drawings and detailed description provided below.

### BRIEF DESCRIPTION OF THE DRAWINGS

FIG. 1 is a front perspective view of the implant in accordance with one embodiment of the present invention.
FIG. 2 is a rear perspective view of the implant shown in FIG. 1.
FIG. 3 is a bottom perspective view of the implant shown in FIG. 1.
FIG. 4 is a bottom perspective view of the implant shown in FIG. 1.
FIG. 5 is a perspective view of the implant shown in FIG. 1 further showing fasteners for fixing the implant.
FIG. 6A illustrates a cross section of a vertebra.
FIG. 6B illustrates a cross section of a vertebra wherein a lamina having an edge is separated from a lateral mass of a vertebra during a laminoplasty procedure.
FIG. 6C illustrates a cross section of a vertebra wherein a lamina having an edge is separated from a lateral mass of a vertebra during a laminoplasty procedure and wherein the first portion of the implant is fixed to the lateral mass.
FIG. 6D illustrates a view of the implant shown in FIGS. 7A-7B.
FIG. 6E illustrates a view of the implant shown in FIGS. 7A-7C, wherein the second portion if fixed to the lamina and the edge of the lamina is received in the receiving section.

### DETAILED DESCRIPTION

The present disclosure describes aspects of the present invention with reference to the exemplary embodiments illustrated in the drawings; however, aspects of the present invention are not limited to the exemplary embodiments illustrated in the drawings. It will be apparent to those of ordinary skill in the art that aspects of the present invention include many more embodiments. Accordingly, aspects of the present invention are not to be restricted in light of the exemplary embodiments illustrated in the drawings. It will also be apparent to those of ordinary skill in the art that variations and modifications can be made without departing from the true scope of the present disclosure. For example, in some instances, one or more features disclosed in connection with one embodiment can be used alone or in combination with one or more features of one or more other embodiments.

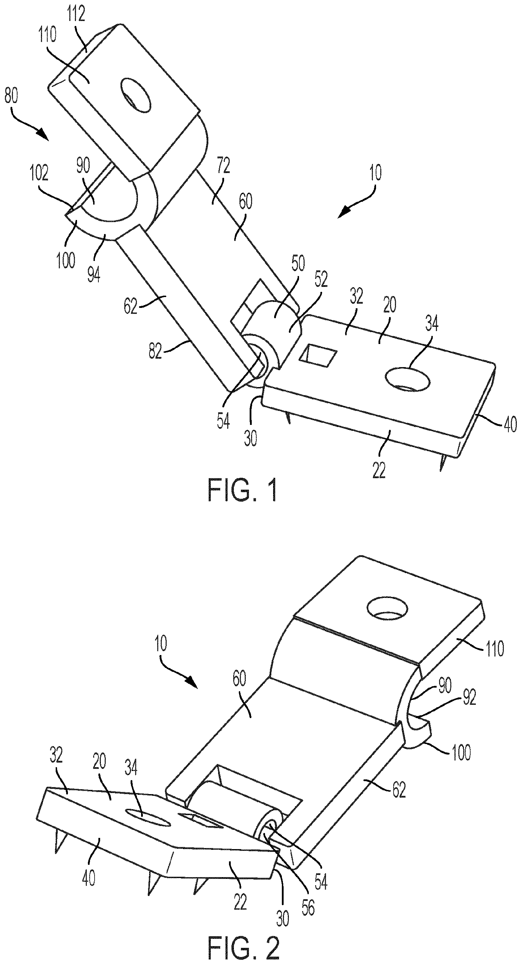

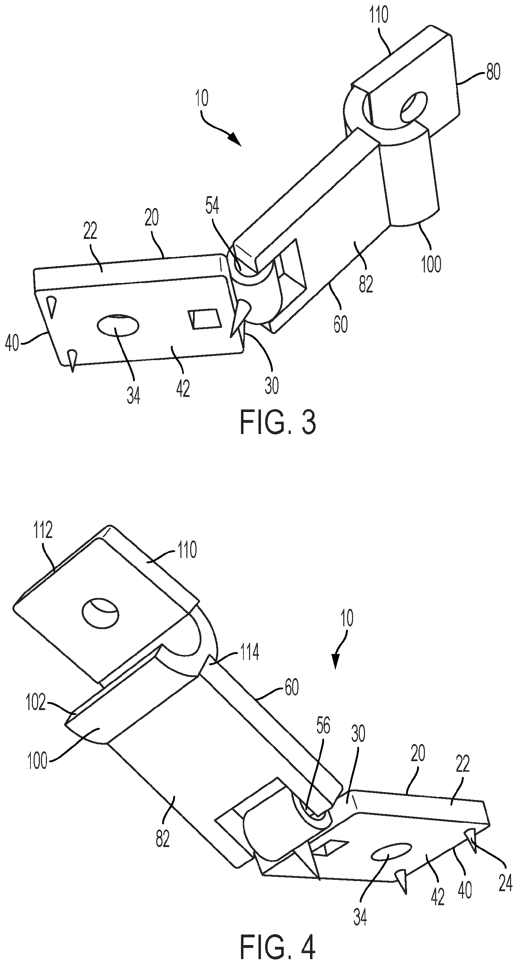

In reference to FIGS. 1-4, an implant 10 for connecting a lamina 130 having an edge 160 separated from a lateral mass 120 of a vertebra 140 during a laminoplasty procedure is shown. The implant 10 includes a first portion 20 configured to engage with the lateral mass 120 (not shown in FIGS. 1-4). The implant 10 includes a second portion 60 configured to engage with the edge 160 of the lamina separated from the lateral mass 120 during the laminoplasty procedure.

In the embodiment disclosed in FIG. 1, the first portion extends 20 in a plane between a first end 30 and a second end 40. The first portion 20 includes a top surface 32 and a bottom surface 42. The bottom surface 44 is configured to interface with the lateral mass 120 when the implant 10, and more specifically the first portion 20, is positioned relative to the lateral mass 120. The first portion 20 defines an edge 22 that extends along the periphery of the first portion 20 between the top surface 32 and the bottom surface 42. In the embodiment disclosed in FIGS. 1-4, the edge 22 of the first portion 20 defines a quadrilateral, although the present invention is not limited in this regard. A person of ordinary skill in the art and familiar with this disclosure with understand that the shape of the first portion may be varied. It is preferred that the bottom surface 44 of the first portion is configured to interface with the lateral mass 120.

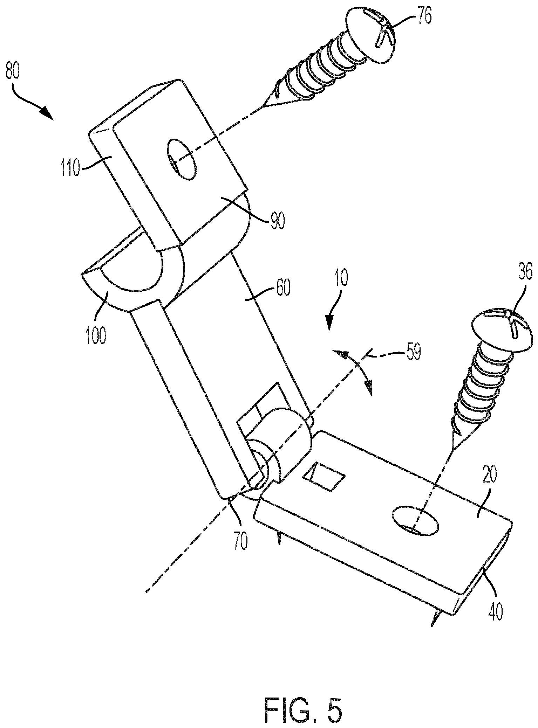

The first portion 20 includes a hole 34 extending between the top surface 32 and the bottom surface 42. The hole 34 is configured for receiving a fixation device such as a bone screw 36 (shown in FIG. 5). The embodiment shown in the FIGS. includes one hole 34 in the first portion 20, however, the present invention is not limited in this regard. A person or ordinary skill in the art will understand that the first portion 10 may include more through holes for receiving bone fixation devices. In the embodiment disclosed in the FIGS., the first portion 20 includes a second hole 44 that extends between the top surface 32 and the bottom surface 42. The second hole 44 is configured to receive surgical instruments for manipulating the implant 10 during the surgical introduction thereof. It should be understood that a second hole 44 is not required to practice the present invention. For example, the second hole 44 may be omitted from the implant 10.

The implant 10 includes a second portion 60 that extends from a proximal end 70 and a distal end 80. The second portion 60 includes a top surface 72 and a bottom surface 82. The second portion 60 defines an edge 62 that extends along the periphery of the second portion 60 between the top surface 72 and the bottom surface 82. In the embodiment disclosed in FIGS. 1-4, the edge 22 of the second portion 60 defines a quadrilateral, although the present invention is not limited in this regard. A person of ordinary skill in the art and familiar with this disclosure with understand that the shape of the second portion 60 may be varied.

The first portion 20 is connected to the second portion 60 so that the first portion 20 is rotatable relative to the second portion 60 about an axis of rotation 59. In the embodiment shown in FIG. 1, the first end 30 of the first portion 20 is connected to the proximal end 70 of the second portion 60. In this manner, the first portion 20 can rotate relative to the second portion 60 about the axis of rotation 59. In the embodiment disclosed in the FIGS. the first portion 20 is connected to the second portion 60 via a hinge 50. In the embodiment disclosed, a portion of the hinge 50 is integral with the first portion 20 and a portion of the hinge is integral with the second portion 60. The first portion 20 includes a knuckle 52 having a bore 54. The knuckle 52 extends from the first end 22 of the first portion 20. The second portion 60 includes two pins 56. The pins 56 are configured to be received in opposing openings 58 in the bore 54 of the knuckle 52. In this manner, the hinge 50 facilitates rotation of the first portion 20 relative to the second portion 60 about the axis of rotation 59. In this embodiment, the pins 56 and the bore 58 are coaxial with the axis of rotation 59 when the hinge 50 is assembled. In this configuration, the hinge 50 can be assembled by the surgical team by snapping the pins 56 into the bore 56. Alternatively, the hinge can be assembled by the manufacturer.

It should be understood that although one embodiment of a hinge is disclosed in the FIGS., the present invention is not limited in this regard and many different techniques may be used for providing rotation of the first portion 20 relative to the second portion 60. For example, in one embodiment of the present invention, the first portion 20 and the second portion may be connected by a flexible material that extends between the first end 30 of the first portion 20 and the proximal end 70 of the second portion 60. In this manner, the material disposed between the first portion 20 and the second portion 60 enables rotation of the first portion 20 relative to the second portion 60. Likewise, many different types of hinges may be used to achieve the rotation.

The second portion 60 includes a hole 74 extending between the top surface 72 and the bottom surface 82. The hole 74 is configured for receiving a fixation device such as a bone screw 76 (shown in FIG. 5). The embodiment shown in the FIGS. includes one hole 74 in the second portion 60, however, the present invention is not limited in this regard. A person of ordinary skill in the art will understand that the second portion 60 may include more than one through hole for receiving bone fixation devices.

In one embodiment of the present invention, the second portion 10 comprises a receiving section 90 configured to receive the edge 160 of the separated lamina. In reference to the embodiment disclosed in FIG. 1-4, the receiving section 90 forms part of the second portion 60 and is at the distal end 80 thereof. In the embodiment disclosed in the FIGS., the receiving section 90 includes an inner member 100 and an outer member 110. The inner member 100 extends distally from the section portion 60 to an inner member distal end 102. The outer member extends distally from the second portion to an outer member distal end 112. As shown in the FIGS., the receiving section 60 defines a space 92 between the inner member 100 and the outer member 110.

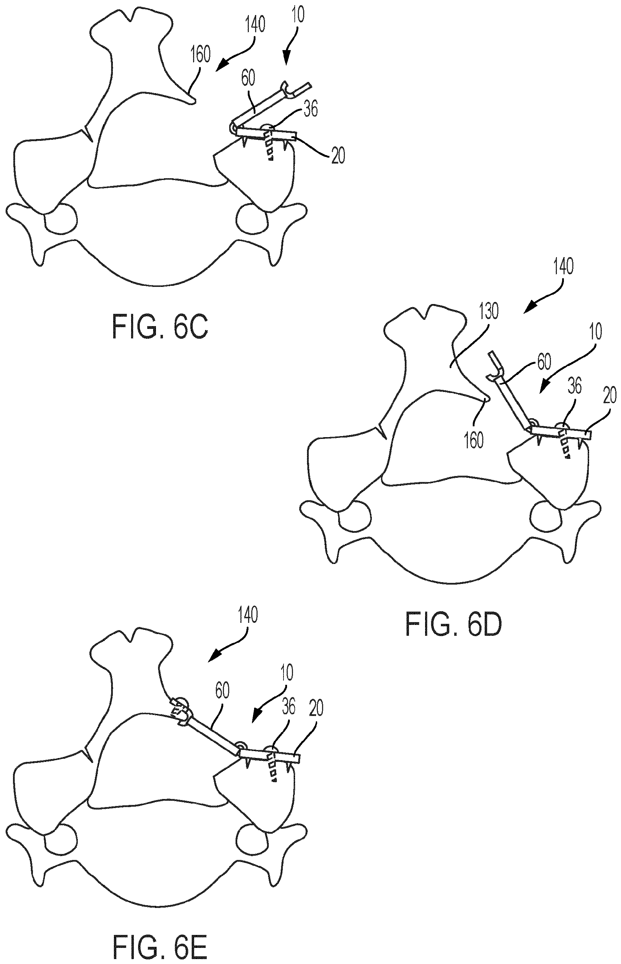

The receiving section 90 is configured to receive the edge 160 of the lamina separated from the lateral mass. This is illustrated, for example, in FIG. 6E. The edge 160 is received in the space 92 between the inner member 100 and the outer member 110. In this manner, the implant 10 in accordance with the present invention facilitates a laminoplasty procedure.

In the embodiments disclosed, the hole 74 in the second portion 60 for receiving the bone screw 76 extends transversely through the outer member 110. In this member, the screw 74 can fix outer member 110, and the second portion 60, to the edge of the lamina 160 separated from the lateral mass.

The receiving section 90 defines a transition 94 between the outer member 110 and the inner member 100. In the embodiment disclosed in the FIGS., the transition is U- shaped in a plane being perpendicular to the second portion 60 and extending from the proximal end 70 to the distal end 80. In this manner, the transition 94 extends along an axis parallel to a vertebral column when the first portion 20 is fixed to the lateral mass 120. It will be understood to a person having ordinary skill in the art and familiar with this disclosure that the present invention is not limited in this regard and different forms of transitions may be used in accordance with the present invention. In some embodiments of the present invention, it may be able to practice the invention without a receiving section. In such embodiments, the first portion 60 is held adjacent to the outer surface of the lamina proximate to the edge 160. A surgical implement may be used to provide an opposing force on the underside of the lamina, while the screw 74 is affixed thereto.

The inventor has discovered that by using the receiving section 90 in accordance with the embodiment of the implant 10 shown in the FIGS., it is possible to provide a stop force on the edge of the lamina, thereby facilitating fixation of the second portion to the lamina via the bone screw. The inventor has discovered that this configuration is particularly advantageous when performing the laminoplasty procedure using minimally invasive techniques or with limited instruments. The implant 10 in accordance with the present invention does not require adjustment at the surgical site to accompany the extent of opening of the lamina. The implant 10 is configured with a range of use of such openings. A further advance of the implant 10 in accordance with the present invention is that it can be more easily fixed to the lateral mass and separated edge.

The distance between the outer member 110 and the inner member 100 is selected so that it is greater than a width of the lamina as determined at the edge thereof. In this manner, the edge of the lamina 160 can be received in the receiving section 90. The inner member 100 is configured to abut an inside surface 162 of the lamina and the outer member 110 is configured to abut an outside 164 surface of the lamina edge 160 when the edge of the lamina is received in the receiving section 90. In some embodiments of the present invention, the inner member 100, the transition section 94, and the outer member 110 define a U-shaped configuration for receiving the edge of the lamina.

In reference to the embodiments disclosed in the FIGS, the outer member 110 extends distally further than the inner member 100. In this manner, the fixation device being receiving through the outer member 110 does not interfere with the inner member 100 to the extent that the screw extends through the lamina. A person of ordinary skill in the art and familiar with this disclosure with understand that the distal extent of the inner member 100 and the outer member 110 member may vary and that the present invention is not limited in this regard. For example, the distal extent of the inner member and the outer member may be the same. In another embodiment, the distal extent of the inner member is greater than the outer member.

In one embodiment of the present invention, the outer member 110 comprises a flexible portion 114 extending in the direction of the vertebral column. The flexible portion 114 is positioned between the transverse opening 74 in the second portion 60 and the proximal end 70 of the second portion. In some embodiments, the flexible portion 114 is provided between the receiving section 90 and the proximal end 70 of the second potion 60. In this manner, the flexible portion 114 provides flexibility in addition to the axis of rotation thereby ensuring that the receiving section interfaces with the lamina properly for the desired fixation thereof.

In one embodiment, the implant 10 is made from titanium. In another embodiment, the implant is made from a biocompatible polymer. In alternate embodiments, the implant 10 any suitable material known to those skill in the art. The implant 10 may further comprise a bone fusion material disposed on one or more surfaces of the implant that interface with the bone. The bone fusion material is selected to encourage osteointegration between the bone and the bone fusion material.

In some embodiments of the present invention, as shown in the FIGSs, the first portion 20 comprises a plurality of positioning pins 24 extending from the interface surface (bottom surface) 42 of the first portion. The positioning pins 24 are configured to engage with the bone an inhibit movement between the first portion 20 relative to the lateral mass 120 while the fastener 36 is installed. In yet other embodiments of the present invention, the positioning pins are provided on the second portion. It will be understood by a person or ordinary skill in the art that the present invention is not limited in this regard, and may be practiced without the positioning pins.

The first portion 20 and the axis of rotation 59 are configured so that the first portion 20 can be fixed to the lateral mass 120 prior to cutting the lamina adjacent thereto. It will be understood to a person of ordinary skill in the art and familiar with this disclosure that the present invention is not limited in this regard and that the lamina may be cut and separated from the lateral mass prior to fixation of the first portion. The inventor has discovered, however, that providing an implant configured for fixation of the first portion 20 prior to separation of the lamina provides several benefits. First, it may simplify the laminoplasty procedure. Second, the implant may provide a guide, either optically or physically, to enable cutting of the lamina in a manner to enable reception of the edge 160 in the receiving section.

In reference to FIG. 5, bone screws 36, 76 for use with the implant 10 of the present invention are disclosed. It should be understood to a person of ordinary skill in the art and familiar with the invention that many different types of bone fixation devices may be used with the present invention. The present invention is not limited to the type of fixation device or the type of bone screw illustrated in FIG. 5.

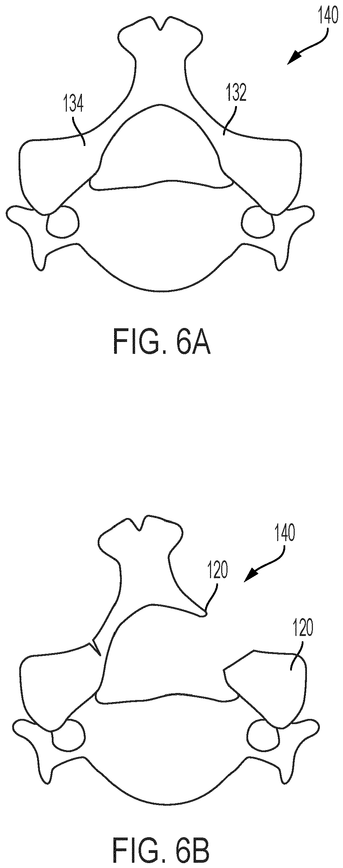

In reference to FIG. 6A-6E, a method for installing the implant 10 in accordance with the present invention is illustrated. FIG. 6A illustrates a cross section of the lamina 130. During the laminoplasty procedure the lamina 130 is repositioned to increase the cavity of the in the cervical section of the spine, thereby decreasing pressure and stress and pressure on spinal nerves disposed therein. The lamina is cut fully through on the first side 132 using a surgical tool. On the opposing side, the lamina may be partially cut, thereby facilitating opening of the lamina 130 as shown in FIG. 6B.

In reference to FIG. 6C, the first portion 20 of the implant is fixed to the lateral mass 120. The bottom surface 42 of the first portion 20 interfaces with the outside surface of the lateral mass. The screw 76 is received through the first portion 20 so that the first portion is fixed to the lateral mass 120. In the embodiment shown, the lamina on the first side 132 is separated and opened so as to form the edge of the lamina 160. This is in the rotated position. The first side 132 of the lamina 130 can be cut prior to fixation of the first portion 20 or after fixation of the first portion.

In reference to FIG. 6D, the second potion 60 is rotated about the axis of rotation 59 so that the receiving section 90 is moved in close proximity to the edge of the lamina 160. The edge 160 and the receiving section are both rotated so that the edge is receiving section 90 between the inner member 100 and the outer member 110. The receiving section 90, and the opposing inner and outer member 100, 110 serve to retain the position of the edge 160 therein while the fixation device 76 is inserted into the lamina, thereby fixing the second potion 60 relative to the edge of the lamina. In this manner, the inventor has discovered that the laminoplasty can be performed more easily and more efficiently as compared to other known methods and systems.

It will be understood to a person of ordinary skill in the art that while a specific embodiment of the implant 10 is shown in the FIGS., the invention is not limited to the specific or relative dimensions of the individual components disclose therein. A person of skill will understand that the dimensions of the implant may vary based on anatomy and the type of the procedure. Furthermore, the FIGS provided herein are intended to disclose the invention.

The inventor has further discovered that a further advantage of the present invention is that the first portion can be rotated relative to the second portion so that the implant is completely collapsed. In this manner, the implant is more company and can inserted to the surgical site through a minimally invasive surgical instrument.

Certain terminology is used herein for purposes of reference only, and thus is not intended to be limiting. For example, terms such as "upper", "lower", "above", and "below" refer to directions in the drawings to which reference is made. Terms such as "front", "back", "rear", "bottom" and "side", describe the orientation of portions of the component within a consistent but arbitrary frame of reference which is made clear by reference to the text and the associated drawings describing the component under discussion. Such terminology may include the words specifically mentioned above, derivatives thereof, and words of similar import. Similarly, the terms "first", "second" and other such numerical terms referring to structures do not imply a sequence or order unless clearly indicated by the context.

When introducing elements or features of the present disclosure and the exemplary embodiments, the articles "a", "an", "the" and "said" are intended to mean that there are one or more of such elements or features. The terms "comprising", "including" and "having" are intended to be inclusive and mean that there may be additional elements or features other than those specifically noted. It is further to be understood that the method steps, processes, and operations described herein are not to be construed as necessarily requiring their performance in the particular order discussed or illustrated, unless specifically identified as an order of performance. It is also to be understood that additional or alternative steps may be employed.

It is specifically intended that the present invention not be limited to the embodiments and illustrations contained herein and the claims should be understood to include modified forms of those embodiments including portions of the embodiments and combinations of elements of different embodiments as come within the scope of the following claims. All of the publications described herein, including patents and non-patent publications are hereby incorporated herein by reference in their entireties.

## Claims

1. An implant (10) for connecting a lamina (130) having an edge (160) separated from a lateral mass (120) of a vertebra (140) during a laminoplasty procedure, the implant comprising:
a first portion (20) configured to engage with the lateral mass (120), the first portion defining a surface (42) for interfacing with the lateral mass (120), the first portion (20) defining a transverse opening (34) for receiving a fastener for fixing the first portion to the lateral mass;
a second portion (60) configured to engage with the edge (160) of the lamina (130) separated from the lateral mass (120) during the laminoplasty procedure, the second portion extending between a proximal end (70) and a distal end (80), the proximal end of the second portion (60) being connected to the first portion (20) so that the second portion is rotatable relative to the first portion about an axis of rotation (59),
wherein the second portion (60) defines a transverse opening (74) for receiving a fastener (76) for fixing the second portion to the lamina (130).

2. The implant (10) of claim 1, wherein the second portion (60) comprises a receiving section (90) configured to receive the edge (160) of the separated lamina (130).

3. The implant (10) of claim 2, the receiving section (90) comprising:
an outer member (110) extending distally from the second portion (60);
an inner member (100) extending distally from the second portion;
the receiving section (90) defining a space between the outer member and the inner member.

4. The implant (10) of claim 3, wherein the receiving section (90) defines a transition (94) between the outer member (110) and the inner member (100), the transition extending along an axis parallel to a vertebral column when the first portion (20) is fixed to the lateral mass (120).

5. The implant (10) of claim 6, wherein a distance between the outer member (110) and the inner member (100) is greater than a width of the lamina (130) at determined at the edge (160) thereof.

6. The implant (10) of claim 5, wherein the inner member (100) is configured to abut an inside surface (162) of the lamina (130) and the outer member (110) is configured to abut an outside surface 164) of the lamina when the edge (160) of the lamina is received in the receiving section (90).

7. The implant (10) of claim 6, wherein the inner member (100) and the outer member (110) define a U-shaped configuration for receiving the edge (160) of the lamina (130).

8. The implant (10) of claim 6, wherein the outer member (110) extends from the second portion (60) further than the inner member (100).

9. The implant (10) of claim 3, wherein the outer member (110) comprises a flexible portion (114) extending in the direction of the vertebral column, the flexible portion positioned between the transverse opening (74) in the second portion (60) and the proximal end (70) of the second portion.

10. The implant (10) of claim 3, further comprising a bone fusion material disposed on one or more surfaces of the implant that interface with the bone, the bone fusion material selected to encourage osteointegration between the bone and the bone fusion material.

11. The implant (10) of claim 3, wherein the first portion (20) comprises a plurality of positioning pins (24) extending from the interface surface (42), the positioning pins configured to engage with the bone an inhibit movement between the first portion (20) relative to the lateral mass (120) while the fastener (76) is installed.

12. The implant (10) of claim 3, wherein the first portion (20) and the axis of rotation (59) are configured so that the first portion can be fixed to the lateral mass (120) prior to cutting the lamina (130) adjacent thereto.

13. The implant (10) of claim 3, wherein the axis of rotation (59) extends along an axis parallel to a vertebral column when the first portion (20) is fixed to the lateral mass (120).

14. The implant (10) of claim 13, wherein the implant comprises a hinge (50) between the first portion (20) and the second portion (60), thereby facilitating rotation of the first portion and the second portion.

15. The implant (10) of claim 3, wherein the receiving section (90) is U-shaped.
